Europäisches Patentamt

European Patent Office

Office européen des brevets

(11) Publication number: **0 492 397 A1**

(19)

(12) **EUROPEAN PATENT APPLICATION**

(21) Application number: **91121698.4**

(22) Date of filing: **18.12.91**

(51) Int. Cl.⁵: **C07H 15/04**

(30) Priority: **26.12.90 JP 406609/90**
     **26.12.90 JP 406610/90**

(43) Date of publication of application:
     **01.07.92 Bulletin 92/27**

(84) Designated Contracting States:
     **DE ES FR NL**

(71) Applicant: **KAO CORPORATION**
     **14-10, Nihonbashi Kayabacho 1-chome**
     **Chuo-ku Tokyo(JP)**

(72) Inventor: **Oka, Hiroshi**
     **Kiwaryo, 4-1, Kinryujicho**
     **Wakayama-shi, Wakayama(JP)**
     Inventor: **Aimono, Kiyoshi**
     **98-12, Nishinosho**
     **Wakayama-shi, Wakayama(JP)**
     Inventor: **Tsuyutani, Shinji**
     **Seiwaryo, 1130, Nishihama**
     **Wakayama-shi, Wakayama(JP)**
     Inventor: **Fujita, Tadaaki**
     **724-124, Zenmyoji**
     **Wakayama-shi, Wakayama(JP)**
     Inventor: **Hashiba, Kunizo**
     **845-108, Ohtani**
     **Wakayama-shi, Wakayama(JP)**

(74) Representative: **Hansen, Bernd, Dr.**
     **Dipl.-Chem. et al**
     **Hoffmann, Eitle & Partner Patent- und**
     **Rechtsanwälte Arabellastrasse 4 Postfach**
     **81 04 20**
     **W-8000 München 81(DE)**

(54) Process for producing alkyl glycoside.

(57) A process for producing an alkyl glycoside from a saccharide and a higher alcohol wherein a saccharide is reacted with a higher alcohol in the presence of an acid catalyst, the solid unreacted saccharide is separated from the reaction mixture to obtain a clear reaction liquor and the clear reaction liquor is aged is disclosed.

A process for producing an alkyl glycoside from a saccharide and a higher alcohol wherein a saccharide is reacted with a higher alcohol in the presence of an acid catalyst, the resulting reaction mixture is aged while the unreacted higher alcohol is distilled off so as to be specified higher alcohol content, the reaction is terminated and the unreacted higher alcohol is further distilled off so as to be specified higher alcohol content is disclosed.

The alkyl glycosides obtained by the present invention have high quality such as an excellent hue and less odor than conventional alkyl glycoside.

EP 0 492 397 A1

Field of the Invention

The present invention relates to a process for producing an alkyl glycoside.

In particular, the invention relates to a process for producing an alkyl glycoside having an excellent hue and an excellent tone of color at a high production efficiency.

The invention, in particular, relates to a process for producing an alkyl glycoside useful as a detergent having a reduced unreacted higher alcohol content.

Description of the Related Art

It is known that alkyl glycosides which are saccharide derivative surfactants have low irritating properties and that even though they are nonionic surfactants, they per se form stable foams and act as a foam stabilizer for other anionic surfactants. Thus they have been attracting attention in recent years as surfactants.

Although the alkyl glycosides have desirable surfactant properties as described above, the practical production of them is quite difficult.

The alkyl glycoside is produced by reacting a saccharide with a higher alcohol. The most serious problem in the production of the alkyl glycoside is that their hue and odor readily deteriorate by various operations in the production step.

Experiments have been conducted for the purpose of preventing the deterioration of the hue in the production step and some processes therefor have been proposed. They include a process wherein a higher alcohol is reacted with a monosaccharide in the presence of an acid catalyst composition comprising an acid catalyst and a reducing agent to obtain an alkyl glycoside as described in U.S.P. No. 4923976; a process wherein a higher alcohol is reacted with a monosaccharide in the presence of an anionic surfactant in acid form as a catalyst to obtain an alkyl glycoside as described in European Patent No. 0132043; and a process wherein neutralization is conducted with an organic base for terminating the reaction as described in European Patent No. 0132046 and U.S.P. No. 4713447. Another process proposed heretofore comprises the addition of a viscosity depressant in the step of separating the formed alkyl glycoside from the recovered unreacted alcohol by distillation, since the deterioration of hue is serious due to a high viscosity and poor heat stability of the alkyl glycoside (see U.S.P. No. 4889925). However, the hue of the alkyl glycoside obtained by any of these processes is still unsatisfactory.

In U.S.P. No. 3565885, it is described that an alkyl glycoside having an excellent hue can be obtained by bringing the reaction product obtained in the alkyl glycoside production process into contact with a basic anion exchange resin of a hydroxyl group type prior to separation. However, this process is undesirable because a bad smell like that of an amine is generated.

U.S.P. No. 4557729 discloses that an alkyl glycoside obtained as the final product is bleached with hydrogen peroxide and a sulfur dioxide source. However, this process cannot serves as a guide for general procedures, since problems other than that of the hue, such as deterioration of smell and poor storability, are encountered.

U.S.P. No. 4762918 proposes a process wherein a glycoside composition containing a colored humin is brought into contact with hydrogen or a hydrogen source such as sodium borohydride to improve the color of the glycoside composition.

Thus, various processes have been proposed for producing an alkyl glycoside having an excellent hue, but they remain unsatisfactory from the standpoint of both hue and odor, since either the hue of the produced alkyl glycoside is unsatisfactory or the odor is deteriorated.

Meanwhile, there is a problem in the process wherein the saccharide is directly reacted with the alcohol, in that, it is difficult to obtain 100% conversion, because a condensate of a solid unreacted saccharide is formed by water contained in the reaction system, such as that formed by the reaction. Since the solid unreacted saccharide or a condensate thereof causes deterioration in quality, such as coloration, a filter aid is added thereto after the completion of the reaction and resulting filter cake containing solid unreacted saccharide, condensate thereof, alkyl glycoside, unreacted alcohol and filter aid is filtered off and discarded, thus making it difficult to obtain a high yield.

Therefore, European Patent No. 378710 discloses a process which comprises reacting a monosaccharide with an alkyl alcohol, crystallizing the resultant alkyl glycoside by addition of a nonpolar solvent, separating the alkyl glycoside from the mother liquor, and circulating unreacted saccharide and alcohol for reuse in the reaction. However, it is impossible to obtain satisfactory conversion and product quality.

The presence of unreacted higher alcohol in the alkyl glycoside is an important factor contributing to the odor of the alkyl glycoside.

Since the alkyl glycoside is usually produced by reacting a saccharide with at least an equivalent amount of a higher alcohol, the reaction product contains a large amount of an unreacted higher alcohol. Because the odor of the remaining higher alcohol per se makes the smell of the alkyl glycoside-containing product bad, the remaining higher alcohol must be separated and removed from the alkyl glycoside before the alkyl glycoside can be used as a surfactant. The unreacted higher alcohol is desirably removed as much as possible from the reaction product.

Although the unreacted higher alcohol can be separated and removed by a well-known means such as distillation, the alkyl glycoside usually has a high melting point and, therefore, the temperature must be increased when it is transported by pump for separation and removal. On the other hand, the thermal stability of most alkyl glycosides is limited and even if heating treatment is conducted in limited time in the process for producing them, the hue of the product is inclined to be impaired and a glycitol is likely to be formed.

Experiments have been conducted heretofore for the purpose of removing the unreacted higher alcohol without impairing the hue of the alkyl glycoside and some processes therefor have been proposed. They include a process wherein a thin film of the reaction product containing the unreacted higher alcohol is formed and the excess alcohol is evaporated at a relatively high temperature in a short residence time (European Patent Nos. 96917 and 92875; U.S.P. No. 4393203); a process wherein the unreacted higher alcohol is distilled off in two steps, the first step being conducted in a thin layer evaporator to reduce the alcohol content to about 40 to 20% by weight while the second step being conducted in a short-path evaporator to reduce the alcohol content to 3 to 5% by weight (European Patent No. 301298); a process wherein an excess unreacted higher alcohol is evaporated by improving the fluidity at a lower temperature in the presence of, if necessary, a small amount of a short-chain alkyl glycoside having 1 to 5 carbon atoms (U.S.P. No. 4393203 and European Patent No. 92875); and a process wherein one or more viscosity depressants having a boiling point higher than that of the unreacted higher alcohol contained therein by at least 50°C under a pressure of 760 millibar and having an activity suitable for use as a detergent, emulsifier, detergent accelerator or the like are added so that the excess unreacted higher alcohol is removed by distillation at a temperature lower than the decomposition temperature of the alkyl glycoside by at least 20°C (U.S.P. No. 4889925).

It is described in the specifications of the above-mentioned patent that it is desirable to use a thin film evaporator in which the residence time is short and in which the material that is treated in the form of a thin film is affected a little by heating treatment. One particular evaporator mentioned is the scraping type evaporator wherein a thin film is forceably formed, in order to inhibit the coloration of the alkyl glycoside at the high temperature used in the distillation of the unreacted higher alcohol, necessitated by the alkyl glycoside having a high melting point.

The distillation of the unreacted higher alcohol is usually conducted in a high vacuum of several Torr and below, since the vapor pressure of the alcohol is quite low and the coloration of the alkyl glycoside due to the heat instability must be inhibited.

In the distillation of the unreacted higher alcohol under these conditions or in the evaporation of the alcohol with a thin film evaporator in a high vacuum, a wiped film evaporator (mfd. by Shinko Pantec Co., Ltd.) or the like is used as the thin film evaporator. The wiped film evaporator has a vapor condenser which is placed close to the evaporation surface of the vapor in the evaporator in order to minimize the pressure drop from the thin film evaporator to a vacuum pump, particularly from the evaporation surface to the condenser.

However, even if, in this case, a mist separator or the like is provided, saccharides such as the alkyl glycoside become inevitably entrained with and contaminate the thus distilled higher alcohol (hereinafter referred to as "distilled alcohol"). Further even when the alcohol is distilled off in two steps as described in European Patent No. 301298, the saccharide is entrained with the distilled alcohol after reducing the content of the unreacted higher alcohol to 20% by weight in the first step and, therefore, the quality of the distilled alcohol is not high.

Because this distilled alcohol is generally used again for the production of the alkyl glycoside, the contamination of the alkyl glycoside with the distilled alcohol creates the same serious problem as that found in the contamination of the unreacted higher alcohol with the alkyl glycoside. When the alkyl glycoside is produced by using the distilled alcohol containing such an impurity even in a small amount, undesirable results such as an impaired hue of the alkyl glycoside, a reduction in the reaction velocity in the production of the alkyl glycoside and a prolongation of the reaction time are encountered. Thus, the distilled alcohol must be purified by some method in order to reuse it as the starting alcohol for the alkyl glycoside. Therefore, in the above-described conventional processes, when an excess amount of the higher alcohol is used for the production of the alkyl glycoside, only fresh alcohol is used, without reusing the distilled alcohol, even though a large amount of the unreacted higher alcohol remains in the reaction product.

Meanwhile, the equipment cost per unit heat-transfer area of the above-described thin film evaporator wherein the liquid is forcedly formed into a thin film and evaporated, is higher than that where other evaporators are used. A large thin-film evaporator having a large heat transfer area is essential for removing the unreacted higher alcohol in one step, otherwise two or more evaporators are needed, thereby requiring an enormous plant investment.

Thus, it has been necessary to develop a process for efficiently removing the excess unreacted higher alcohol from the reaction product and a process for producing the alkyl glycoside wherein the distilled alcohol thus obtained has a quality equal to that of a fresh higher alcohol and is reusable as is, without necessitating any further treatment.

Summary of the Invention

After extensive investigations made for the purpose of solving the above-described problem, the inventors have found that the deterioration of the hue of the alkyl glycoside is mainly caused by a coloring substance such as a dissolved unreacted saccharide, and an acid component and a furan derivative formed by the decomposition of the unreacted saccharide. The inventors have discovered that when the solid unreacted saccharide is separated out from the reaction mixture, the amount of the dissolved unreacted saccharide is rapidly reduced and an alkyl glycoside of a high quality can thus be obtained, and the yield can also be increased by reusing the solid unreacted saccharide separated as described above. The present invention has been completed on the basis of this discovery.

Thus, the first embodiment of the present invention provides a process for producing an alkyl glycoside from a saccharide and a higher alcohol comprising the steps of; (1) reacting the saccharide with the higher alcohol in the presence of an acid catalyst to produce an alkyl glycoside, (2-a) separating off solid unreacted saccharide from the reaction mixture containing the alkyl glycoside to obtain a clear reaction liquor and (3-a) aging and reacting the clear reaction liquor.

The (3-a) step is conducted so that the amount of a unreacted saccharide dissolved in the clear reaction liquor is 500 ppm and less is preferable.

The (3-a) step is preferably conducted in a thin film state and more preferably conducted with a falling film reactor, a climbing film reactor, a wiped film reactor or a drum evaporator.

The (3-a) step is preferably conducted at a temperature of 60 to 130°C.

The (2-a) step is conducted in a continuous manner is preferable.

Furthermore, the (2-a) step uses a filter such as a cross-flow filter, a pressure filter and a vacuum filter preferably.

According to the process of the first embodiment of the present invention, an alkyl glycoside of a high quality can be produced because the amount of unreacted saccharide which is dissolved in the reaction mixture containing the alkyl glycoside and which causes the coloration is 500 ppm and less. Additionally, the solid unreacted saccharide thus separated can be reused for the initial reaction.

Furthermore, the inventors have found a process for producing an alkyl glycoside wherein the excess unreacted higher alcohol can be efficiently removed by evaporation from a reaction mixture containing the alkyl glycoside while the heating is kept as low as possible. The higher alcohol thus removed has a quality sufficient for reuse for the production of the alkyl glycoside with the plant investment being kept low. The present invention has been completed on the basis of this discovery.

Thus, the second embodiment of the present invention provides a process for producing an alkyl glycoside from a saccharide and a higher alcohol comprising the steps of; (1) reacting the saccharide with the higher alcohol in the presence of an acid catalyst to produce an alkyl glycoside, (2-b) aging and reacting the reaction mixture while distilling off unreacted higher alcohol from the reaction mixture so as to adjust the concentration of the higher alcohol to 30 to 60% by weight, (3-b) terminating the reaction by neutralizing the catalyst and (4-b) further distilling off unreacted higher alcohol so as to reduce the concentration of the higher alcohol to 5% by weight and less.

The (2-b) step is preferably conducted at a temperature of 60 to 130°C.

The (2-b) step is conducted with a thin film reactor such as a falling film reactor, a climbing film reactor, a wiped film reactor and a drum evaporator preferably.

The neutralization is preferably conducted with an alkali metal hydroxide.

The (4-b) step is preferably conducted in a thin film state.

In the above-described first and second embodiments of present invention, the saccharide is preferably selected from the group consisting of monosaccharides, oligosaccharides and polysaccharides, more preferably glucose.

4

In the above-described first and second embodiments of present invention, the higher alcohol is preferably an alkyl alcohol having 8 to 22 carbon atoms or an alkylene oxide adduct of said alkyl alcohol, more preferably n-decylalcohol.

In the above-described first and second embodiments of present invention, 2 to 10 mols of the higher alcohol is used per mol of saccharide is preferable.

Detailed Description of the Invention

The acid catalysts usable for the reaction of the saccharide with the higher alcohol in the present invention include, p-toluenesulfonic acid, sulfuric acid, phosphoric acid and strongly acidic ion exchange resins. The amount of the acid catalyst used is preferably 0.001 to 0.10 mol per mol of the saccharide. With the acid catalyst in an amount below this range, the reaction velocity is seriously lowered and, on the contrary, when the amount exceeds this range, the hue of the alkyl glycoside becomes bad.

The saccharides to be used as the starting material in the process of the present invention include monosaccharides, oligosaccharides and polysaccharides. Examples of the monosaccharides include aldoses such as allose, altrose, glucose, mannose, gulose, idose, galactose, talose, ribose, arabinose, xylose and lyxose. Examples of the oligosaccharides include maltose, lactose, sucrose and maltotriose. Examples of the polysaccharides include hemicellulose, inulin, dextrin, dextran, xylan, starch and hydrolyzed starch. Among them, reducing sugars having 6 and less carbon atoms are desirable.

The higher alcohols to be used as the starting material in the process of the present invention are preferably straight-chain or branched, saturated or unsaturated alcohols having 8 to 22 carbon atoms and alkylene oxide adducts thereof.

As for the relative amounts of the starting materials, 2 to 10 mols of the higher alcohol is preferably used per mol of the saccharide. When the amount of the higher alcohol is below this range, the conversion is insufficient and, on the other hand, even when it exceeds this range, no advantages can be obtained from a technical or economic standpoint.

The reaction of the saccharide with the higher alcohol may be conducted either batchwise or continuously in the present invention. The reactors usable herein include a stirring tank for either batchwise or continuous reaction, a suspension bubble column and a tubular reactor. The reaction temperature is preferably 70 to 140°C, and more preferably 90 to 120°C. When the reaction temperature is above 140°C, the deterioration of the hue is serious and a condensate of an unreacted saccharide is formed in a large amount which impairs the quality. On the other hand, when the reaction temperature is below 70°C, the reaction velocity is greatly reduced.

According to the present invention, two kinds of treatment methods for the reaction mixture produced by the above-described reaction (1) are provided.

In the first embodiment of the present invention, a step of separating off a solid unreacted saccharide from the reaction mixture containing the alkyl glycoside to obtain a clear reaction liquor (2-a) and a step of aging and reacting the clear reaction liquor (3-a) are performed.

The (2-a) step can be conducted either batchwise or continuously. Separated unreacted saccharide is used again for the reaction with the higher alcohol. The conversion ratio at separating the solid unreacted saccharide is determined depending on the molar ratio of the starting materials used and a desired average degree of condensation of the alkyl glycoside (average number of the condensed saccharide molecules per molecule of the higher alcohol). The relationship between the molar ratio of the starting materials used and the average degree of condensation and conversion ratio is as given in Table 1. The conversion ratio of saccharide in the Table 1 is expressed in the consumption of solid saccharide.

Table 1

| Molar feed ratio (higher alcohol/ saccharide) | Conversion | | |
|---|---|---|---|
| | average degree of condensation | | |
| | 1.2 | 1.4 | 1.6 |
| 2 | 25% | 50% | 70% |
| 3 | 30% | 70% | 95% |
| 5 | 60% | 98% | — |

In the (2-a) step, a filter and a centrifugal separator are used. The filters usable herein include a cross flow filter (with an inorganic or organic membrane, type: hollow module, flat membrane module, etc.), a pressure filter and a vacuum filter. In view of the compressibility of the cake, the cross flow filter is preferred. The centrifugal separators include those of decanter type, separating disc type and basket type.

In the first embodiment of the present invention, the (3-a) step can be conducted either batchwise or continuously. The temperature of the (3-a) step is preferably 60 to 130°C, and more preferably 70 to 110°C. When the temperature exceeds 130°C, the deterioration of the hue is serious and, in addition, the average degree of condensation of the alkyl glycoside is increased to make it difficult to obtain a product having a desired quality. On the other hand, when it is below 60°C, the reaction velocity is low.

The (3-a) step is preferably conducted in a thin film state and the thin film reactors usable herein include a falling film reactor, a climbing film reactor, a wiped film evaporator and a drum evaporator. The thickness of the film which varies depending on the apparatus is 5 mm and less, preferably 2 mm and less.

The following advantages can be obtained by using the above-described thin film reactor:

1) since the aging and reacting step is a rate-determined dehydration, the reaction time can be reduced by using the thin film reactor having a high dehydration efficiency (evaporation efficiency), and

2) thermal coloration can be inhibited, since the thin film reactor has a high heat transfer efficiency and therefore the clear reaction liquor is subjected to heat for a short period.

In the first embodiment of the present invention, the acid catalyst is neutralized and resulting alkyl glycoside is isolated by conventional methods after the (3-a) step.

In the second embodiment of the present invention, after the reaction mixture is obtained as above-described (the (1) step), a step of aging and reacting the reaction mixture while distilling off unreacted higher alcohol from the reaction mixture to adjust the concentration of the higher alcohol to 30 to 60% by weight (2-b), a step of terminating the reaction by neutralizing the catalyst (3-b) and a step of distilling off unreacted higher alcohol to reduce the concentration of the higher alcohol to 5% by weight and less (4-b) are conducted.

The alkyl glycoside is usually produced by reacting a saccharide with at least an equimolar amount of a higher alcohol. When the reaction is substantially completed (the conversion ratio being at least 95%), an unreacted dissolved saccharide still remains therein in an amount of 1000 to 3000 ppm. The reaction must be further continued until the amount of the unreacted dissolved saccharide has been reduced to 500 ppm and less, since the unreacted dissolved saccharide causes the coloration. However, in this step, it is no more necessary that the higher alcohol is excess as compared with the saccharide. Since the reaction is dehydration reaction, the cost in the subsequent step of separating the unreacted higher alcohol can be reduced and the plant investment can be also reduced by distilling off the unreacted higher alcohol together with water formed. In the process of the second embodiment of the present invention, after the reaction mixture is obtained, the aging and reacting of the reaction mixture is conducted while simultaneously the unreacted higher alcohol is distilled off from the reaction mixture so as to adjust the concentration of the higher alcohol to 30 to 60% by weight (2-b), and then the catalyst is neutralized to terminate the reaction (3-b).

The temperature of the (2-b) step is preferably 60 to 130°C, and more preferably 70 to 120°C. When the reaction temperature exceeds 130°C, the deterioration of the hue is serious and, in addition, the average degree of condensation of the alkyl glycoside is increased making it difficult to obtain a product having a desirable quality. On the other hand, when it is below 60°C, the reaction velocity is low, the viscosity of the reaction solution is increased, the heat transfer efficiency is reduced and the evaporation velocity of the unreacted higher alcohol is reduced.

The apparatus used for conducting the (2-b) step may be a stirred tank, but a thin film reactor, having a high evaporation efficiency which permits the reaction mixture to receive low heating treatment and therefore inhibition of thermal coloration is preferred. A suitable thin film reactor is a falling film reactor, a climbing film reactor, a wiped film evaporator and a drum evaporator. The thickness of the film is preferably 5 mm and less, more preferably 2 mm and less.

The concentration of the unreacted higher alcohol in the reaction mixture obtained after distilling off the unreacted higher alcohol in the (2-b) step [(weight of unreacted higher alcohol)/{(weight of alkyl glycoside) + (weight of unreacted higher alcohol)} x 100] is 30 to 60% by weight. When the concentration of the unreacted higher alcohol exceeds 60% by weight, the amount of the unreacted higher alcohol evaporated in the (4-b) step is substantially constant irrespective of the distillation of the unreacted higher alcohol in the (2-b) step. Thus, the advantage of the distillation in the (2-b) step is only slight. On the other hand, when it is less than 30% by weight, the catalyst concentration in the reaction mixture is increased and the hue is easily deteriorated.

Successively, the catalyst is neutralized to terminate the reaction (3-b). The neutralization of the catalyst is conducted by using alkali metal agent such as sodium hydroxide, potassium hydroxide and sodium carbonate.

In the (4-b) step of the present invention, the concentration of the unreacted higher alcohol in the reaction mixture containing alkyl glycoside [(weight of unreacted higher alcohol)/{(weight of alkyl glycoside) + (weight of unreacted higher alcohol)} x 100} comes to 5% by weight and less by distilling off the unreacted higher alcohol. When the concentration of the unreacted higher alcohol exceeds 5% by weight, the quality of the product containing it is deteriorated due to the odor of the unreacted higher alcohol per se.

In the (4-b) step, the temperature of the liquid distillation residue (the alkyl glycoside) which varies depending on the vapor pressure of the alcohol is 100 to 250°C, preferably 120 to 180°C. When the temperature is above this range, the hue of the alkyl glycoside is deteriorated and, when it is below this range, the viscosity of the solution is increased, the heat transfer efficiency is reduced and the evaporation velocity of the alcohol is reduced.

In the (4-b) step of the present invention, the distillation is preferably conducted in a thin film state. The apparatuses used herein include thin film evaporators (such as a falling film reactor, a climbing film reactor, a wiped film evaporator and a drum evaporator).

According to the first embodiment of the present invention, the results described below are obtained.

(1) When the clear reaction liquor obtained by solid-liquid separation is aged and reacted, the velocity of reducing the concentration of the dissolved unreacted saccharide which causes the coloration is increased 4 to 5 times as high as that obtained when the reaction mixture containing the solid unreacted saccharide is directly aged and reacted. The amount of the dissolved unreacted saccharide must be reduced to 500 ppm and less, preferably 100 ppm and less. When the clear reaction liquor obtained by solid-liquid separation is aged and reacted, the amount of the dissolved unreacted saccharide can be reduced to 500 ppm and less in a short period of time. Further, by reducing the aging and reacting time, the coloration due to the aging and reacting can be reduced.

(2) Since the solid unreacted saccharide is reusable, the solid-liquid separation may be conducted when the conversion ratio of saccharide is low. Namely, since the molar ratio of the starting materials can be lowered as shown in the Table 1, the productivity is improved.

(3) Since no solid unreacted saccharide is discharged from the reaction system (i.e. it is reused), the reaction yield becomes close to 100% as the frequency of repetition is increased.

According to the second embodiment of the present invention, the results described below are obtained.

By conducting the aging, reacting and partial removal (distillation) of the unreacted higher alcohol at the same time according to the present invention, the burden in the (4-b) step for the distillation of the unreacted higher alcohol is reduced along with the equipment cost. Further by conducting the distilling of the unreacted higher alcohol and the dehydration simultaneously in the (2-b) step, the dehydration efficiency is improved and the reaction velocity is increased. In other words, the second embodiment of the present invention also has the effect of rapidly reducing the amount of the unreacted dissolved saccharide which causes coloration.

Examples

The following Examples will further illustrate the present invention, but should not be construed to unduly limit the present invention, as modifications and variations in the embodiments herein discussed may be made by those of ordinary skill in the art without departing from the spirit or the scope of the present inventive discovery.

(Example 1)

800.2 g (5 mol) of n-decylalcohol, 180.1 g (1 mol) of anhydrous glucose and 0.67 g ($3.5 \times 10^{-3}$ mol) of p-toluenesulfonic acid were subjected to dehydration reaction in a 2-ℓ stirred tank at a temperature of 110°C under a reaction system pressure of 40 mmHg. During the reaction, 200 ml/min of nitrogen was blown into the liquid reaction mixture in order to efficiently remove water formed by the reaction. After conducting the reaction under heating for 6 hours, the conversion ratio of glucose reached 93.8%. The liquid reaction mixture was filtered through a cross flow filter comprising a porous ceramic membrane having a pore diameter of 1.8 $\mu$m. The filtrate thus obtained was aged and reacted in a falling film reactor having an inner diameter of 25 mm and a tube length of 1000 mm under conditions comprising a circulation rate of 1.0 ℓ/min, a temperature of 90°C and a pressure in the reaction system of 40 mmhg for 2 hours. After the completion of the reaction, the pressure was elevated to the atmospheric pressure and the reaction mixture containing p-toluenesulfonic acid was neutralized with 0.29 g of 48% by weight aqueous NaOH solution. Then 250 g of the formed alkyl glucoside and 660 g of n-decylalcohol were separated under distillation conditions of 160°C and 0.5 mmHg. The resultant alkyl glucoside was dissolved in water to give a 35% by weight aqueous solution thereof for determining the hue.

(Example 2)

104.3 kg (652 mol) of n-decylalcohol, 58.7 kg (326 mol) of anhydrous glucose and 0.22 kg (1.14 mol) of p-toluenesulfonic acid were subjected to dehydration reaction in a 200-ℓ stirred tank of a continuous system reactor at a temperature of 100°C under a reaction system pressure of 40 mmHg. During the reaction, 1.2 m$^3$/hr of nitrogen was blown into the liquid reaction mixture in order to efficiently remove water formed by the reaction. After conducting the reaction under heating for 6 hours, the conversion ratio of glucose reached 40%. Then circulation for filtration was started at a rate of 1200 kg/hr (the stirred tank was connected with a circulation line having a cross flow filter with the separating membrane of the cross flow filter being a porous ceramic membrane having a pore diameter of 1.8 $\mu$m and a filtration area of 0.2 m$^2$). During circulation for filtration was conducted, 22.2 kg/hr of n-decylalcohol, 5 kg/hr of anhydrous glucose and 0.047 kg/hr of p-toluenesulfonic acid were continuously fed into the stirred tank. Unreacted glucose which was contained in a filter cake was put back to the stirred tank for reuse. A clear filtrate which filtrated through the cross flow filter was continuously obtained or discharged at a rate of 26.7 kg/hr.

The obtained filtrate was stored in a 100-ℓ intermediate tank and a part of it was aged and reacted in a falling film reactor having an inner diameter of 43 mm and a tube length of 4 m at a circulation flow rate of 360 kg/hr and a temperature of 90°C under a pressure of the reaction system of 40 mmHg for 1 hour. Namely, the filtrate was continuously fed from the intermediate tank to the falling film reactor at a rate of 26.7 kg/hr and was aged and reacted in the falling film reactor as described above, and resulting reacting mixture was discharged from the falling film reactor at a rate of 26.6 kg/hr and neutralized with 0.021 kg/hr of 48% by weight of aqueous NaOH solution. Thereafter, the formed alkyl glucoside and unreacted n-decylalcohol were isolated under distillation conditions of 160°C and 0.5 mmHg to give 7.9 kg/hr of the alkyl glucoside. Then the alkyl glucoside thus obtained was dissolved in water to obtain a 35% by weight aqueous solution thereof for evaluating the hue.

The alkyl glucoside obtained from the filtrate (1) obtained 2 hours after the circulation was started, that is, 8 hours after the dehydration reaction was started, and the filtrate (2) obtained 54 hours after the circulation was started, were conducted to the evaluation.

(Example 3)

The alkyl glucoside was produced in the same manner as that of the Example 1 except that the aging and reacting was conducted in the stirred tank. The reaction conditions were the same as those of the Example 1.

(Comparative Example 1)

The alkyl glucoside was produced in the same manner as that of the Example 3 except that no filtration was conducted. The reaction conditions were the same as those of the Example 3. The conversion ratio of glucose reached 97.6% before the neutralization was conducted.

Table 2 gives the results of the determination and examination of the amount of the unreacted dissolved saccharide and the hue of the reaction liquor at the time of starting of the aging and reacting; the amount of the unreacted dissolved saccharide and the hue of the reaction liquor after the completion of the aging and reacting; and the hue and odor of the 35% by weight aqueous alkyl glucoside solution obtained after the removal of unreacted n-decylalcohol.

Table 2

| | | Ex.1 | Ex.2 filtrate (1) | Ex.2 filtrate (2) | Ex.3 | Comp. Ex. 1 |
|---|---|---|---|---|---|---|
| At start of aging and reacting - | Amount of unreacted dissolved saccharide hue | 1800 ppm APHA 120 | 2480 ppm APHA 120 | 2200 ppm APHA 120 | 1800 ppm APHA 120 | 1800 ppm APHA 120 |
| Aging and reacting time | | 2 Hr | 1 Hr | 1 Hr | 2 Hr | 2 Hr |
| After completion of aging and reacting* | Amount of unreacted dissolved saccharide hue | 40 ppm APHA 150 | 200 ppm APHA 150 | 210 ppm APHA 150 | 230 ppm APHA 200 | 1050 ppm APHA 200 ~250 |
| After removal of unreacted n-decyl alcohol | hue** odor** | APHA 200 good | APHA 200 good | APHA 200 good | APHA 250 good | APHA 500 good |

Note) *: After completion of neutralization
**: 35% by weight aqueous alkyl glucoside solution

It is apparent from the Table 2 that when unreacted glucose (solid) was separated by filtration from the reaction mixture obtained by reacting the saccharide with the higher alcohol and the obtained filtrate was aged and reacted, the obtained alkyl glucoside had a hue superior to that of the product obtained after the aging and reacting without filtration and separation from unreacted glucose.

Also, when reuse of the separated unreacted glucose (solid) was conducted as shown in Example 2, the alkyl glucoside obtained had a hue superior to that of the product obtained after the aging and reacting without filtration and separation from unreacted glucose. Though the conversion ratio of glucose was 97.6% when the reaction mixture was aged and reacted without filtration and separation from unreacted glucose (See Comp. Ex. 1), 2.4% of anhydrous glucose based on the anhydrous glucose used as a raw material was removed as a filter cake at this case. Meanwhile, in the case of Example 2 in which reuse of the separated unreacted glucose (solid) was conducted, no filter cake was removed, and, as a result, high yield (about 100% based on glucose) was achieved at this case.

(Example 4)

(1) 5 mol of n-decylalcohol was used per mol of anhydrous glucose and 0.0035 mol of p-toluenesulfonic acid monohydrate was used per mol of anhydrous glucose. The reaction was conducted in a stirred tank at a temperature of 110°C under a pressure of 40 mmHg. After 5.0 hours, the conversion ratio of glucose reached 97%. The amount of unreacted dissolved glucose in the reaction mixture was 2000 ppm and the hue value was Gardner No. 1.

(2) The reaction mixture obtained as described above was introduced into a falling film reactor (heat transfer area: 0.079 m$^2$) and, aging and reaction of the reaction mixture and distillation of unreacted n-decylalcohol were conducted at 90°C under 1 mmHg for 30 minutes. The amount of unreacted dissolved glucose in the reaction mixture was 40 ppm and the concentration of unreacted n-decylalcohol was 53.7% by weight. When the concentration of unreacted n-decylalcohol was adjusted to that before the distillation (72.0% by weight), the hue value was Gardner No. 1. The reaction mixture was neutralized with sodium hydroxide.

EP 0 492 397 A1

(3) The reaction mixture, containing alkyl glucoside, obtained in the step (b) was introduced into a Smith thin film evaporator (mfd. by Shinko Pantec Co., Ltd.; heat transfer area: 0.034 m$^2$) at a rate of 300 ml/min and treated at 160°C under 0.3 mmHg. The concentration of an unreacted alcohol in the resulting reaction product mainly containing alkyl glucoside was 0.7% by weight. A 35% by weight aqueous solution of this product had a hue value of Gardner No. 2 to 3.

(Comparative Example 2)

(1) The reaction was conducted in the same manner as that of the Example 4 (1) by using 5 mol of n-decylalcohol per mol of anhydrous glucose and 0.0035 mol of p-toluenesulfonic acid monohydrate per mol of anhydrous glucose. Then, the catalyst was neutralized with sodium hydroxide. The reaction time was as long as 5.0 hours. The conversion ratio of glucose was 97%, the amount of unreacted dissolved glucose was 2000 ppm and the hue value was Gardner No. 1.

(2) The reaction mixture, containing alkyl glucoside, obtained in the step (1) was treated in the same manner as that of the Example 4 (3). The concentration of unreacted n-decylalcohol in the obtained reaction product mainly containing alkyl glucoside was 7.0% by weight. A 35% by weight aqueous solution of this product had a hue value of Gardner No. 5.

(Example 5)

The reaction mixture, containing alkyl glucoside, obtained in the same manner as that of Example 4 (1) and (2) was treated with a falling film evaporator at a circulation flow rate of 2.0 ℓ/min, at 140°C under 0.2 mmHg for 20 minutes. The concentration of an unreacted alcohol in the obtained reaction product mainly containing alkyl glucoside was 0.7% by weight. A 35% by weight aqueous solution of this product had a hue value of Gardner No. 2 to 3.

The reaction conditions and results in the Examples 4 and 5 and Comparative Example 2 are summarized in Table 3.

Table 3

| | | Ex. 4 | Ex. 5 | Comp. Ex. 2 |
|---|---|---|---|---|
| Reaction mixture obtained before neutralization | amt. of unreacted dissolved glucose | 40 ppm | 40 ppm | 2000 ppm |
| | hue | Gardner No. 1[*1] | Gardner No. 1[*1] | Gardner No. 1[*1] |
| | conc. of unreacted n-decylalcohol | 53.7% | 53.7% | 72% |
| Reaction product (Alkyl glucoside) obtained | conc. of unreacted n-decylalcohol | 0.7% | 0.7% | 7.0% |
| | hue (35% aq. soln.) | Gardner No. 2~3 | Gardner No. 2~3 | Gardner No. 5 |
| | odor(35% aq. soln.) | good | good | odor of alcohol |

Note) *1: The concentration of unreacted n-decylalcohol was adjusted to the same level (72%) as that prior to the first step to determine the hue value.

It is apparent from this Table 3 that an alkyl glucoside superior to that obtained by conventional processes from the standpoint of hue and odor could be obtained by the present invention.

**Claims**

**1.** A process for producing an alkyl glycoside from a saccharide and a higher alcohol comprising steps;
(1) reacting the saccharide with the higher alcohol in the presence of an acid catalyst to produce an alkyl glycoside,
(2-a) separating off solid unreacted saccharide from the reaction mixture containing the alkyl glycoside to obtain a clear reaction liquor, and
(3-a) aging and reacting the clear reaction liquor.

10

2. The process according to Claim 1, wherein the (3-a) step is conducted so that the amount of a unreacted saccharide dissolved in the clear reaction liquor is 500 ppm and less.

3. The process according to Claim 1, wherein the (3-a) step is conducted in a thin film state.

4. The process according to Claim 3, wherein the (3-a) step is conducted with a falling film reactor, a climbing film reactor, a wiped film reactor or a drum evaporator.

5. The process according to Claim 1, wherein the (3-a) step is conducted at a temperature of 60 to 130°C.

6. The process according to Claim 1, wherein the (2-a) step is conducted in a continuous manner.

7. The process according to Claim 1, wherein the (2-a) step uses a filter.

8. The process according to Claim 7, wherein the filter is a cross-flow filter, a pressure filter or a vacuum filter.

9. A process for producing an alkyl glycoside from a saccharide and a higher alcohol comprising steps;
    (1) reacting the saccharide with the higher alcohol in the presence of an acid catalyst to produce an alkyl glycoside,
    (2-b) aging and reacting the reaction mixture while distilling off unreacted higher alcohol from the reaction mixture so as to adjust the concentration of the higher alcohol to 30 to 60% by weight,
    (3-b) terminating the reaction by neutralizing the catalyst, and
    (4-b) further distilling off unreacted higher alcohol so as to reduce the concentration of the higher alcohol to 5% by weight and less.

10. The process according to Claim 9, wherein the (2-b) step is conducted at a temperature of 60 to 130°C.

11. The process according to Claim 9, wherein the (2-b) step is conducted with a thin film reactor.

12. The process according to Claim 11, wherein the thin film reactor is a falling film reactor, a climbing film reactor, a wiped film reactor or a drum evaporator.

13. The process according to Claim 9, wherein the neutralization is conducted with an alkali metal hydroxide.

14. The process according to Claim 9, wherein the (4-b) step is conducted in a thin film state.

15. The process according to Claims 1 or 9, wherein the saccharide is selected from the group consisting of monosaccharides, oligosaccharides and polysaccharides.

16. The process according to Claim 15, wherein the saccharide is glucose.

17. The process according to Claims 1 or 9, wherein the higher alcohol is an alkyl alcohol having 8 to 22 carbon atoms or an alkylene oxide adduct of said alkyl alcohol.

18. The process according to Claims 17, wherein the higher alcohol is n-decylalcohol.

19. The process according to Claims 1 or 9, wherein 2 to 10 mols of the higher alcohol is used per mol of saccharide.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | WO-A-9 006 933 (HENKEL KOMMANDITGESELLSCHAFT AUF AKTIEN) <br> * page 9, line 2 - line 28; claims 1-7 * <br> --- | 1-8, 17-19 | C07H15/04 |
| X | US-A-4 950 743 (P.M. MCCURRY, JR. AND C.E. PICKENS) <br> * example 1 * <br> --- | 9,17-19 | |
| A | WO-A-9 008 154 (HENKEL KOMMANDITGESELLSCHAFT AUF AKTIEN) <br> * example 1 * <br> ----- | 9 | |
| | | | **TECHNICAL FIELDS SEARCHED (Int. Cl.5 )** <br><br> C07H |

The present search report has been drawn up for all claims

| Place of search | Date of completion of the search | Examiner |
|---|---|---|
| THE HAGUE | 06 APRIL 1992 | DAY G.J. |

EPO FORM 1503 03.82 (P0401)